(19) Europäisches Patentamt
European Patent Office
Office européen des brevets

(11) EP 4 216 910 B1

(12) EUROPEAN PATENT SPECIFICATION

(45) Date of publication and mention of the grant of the patent:
**17.09.2025 Bulletin 2025/38**

(21) Application number: **21790025.7**

(22) Date of filing: **21.09.2021**

(51) International Patent Classification (IPC):
***A61K 8/31*** (2006.01) ***A61K 8/73*** (2006.01)
***A61K 8/891*** (2006.01) ***A61K 8/894*** (2006.01)
***A61K 8/92*** (2006.01) ***A61Q 1/00*** (2006.01)
***A61Q 19/00*** (2006.01)

(52) Cooperative Patent Classification (CPC):
**A61Q 1/00; A61K 8/31; A61K 8/731; A61K 8/891; A61K 8/894; A61K 8/92; A61Q 19/00**

(86) International application number:
**PCT/US2021/051168**

(87) International publication number:
**WO 2022/066592 (31.03.2022 Gazette 2022/13)**

(54) **SKIN CARE FORMULATION**

HAUTPFLEGEFORMULIERUNG

FORMULATION DE PRODUIT DE SOIN POUR LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.09.2020 US 202063082768 P**

(43) Date of publication of application:
**02.08.2023 Bulletin 2023/31**

(73) Proprietors:
- **Dow Global Technologies LLC**
  **Midland, MI 48674 (US)**
- **Dow Silicones Corporation**
  **Midland, Michigan 48686 (US)**
- **Rohm and Haas Company**
  **Collegeville, PA 19426 (US)**

(72) Inventors:
- **XU, Wenjun**
  **Collegeville, PA 19426 (US)**
- **COURTEMANCHE, Marc-Andre**
  **Midland, MI 48686-0994 (US)**
- **VAN REETH, Isabelle**
  **7180 Seneffe (BE)**
- **DIHANG, Helene**
  **7180 Seneffe (BE)**
- **LAN, Tian**
  **Collegeville, PA 19426 (US)**
- **LEE, Myoungbae**
  **Midland, MI 48686-0994 (US)**
- **MCLAUGHLIN, Matthew**
  **Midland, MI 48686-0994 (US)**

(74) Representative: **Jewell, Catherine Mary**
**Beck Greener LLP**
**Fulwood House**
**12 Fulwood Place**
**London WC1V 6HR (GB)**

(56) References cited:
**WO-A1-2010/058148 JP-B2- 2 953 601**
**US-A1- 2001 021 387**

- **GOUSSE C ET AL: "Surface silylation of cellulose microfibrils: preparation and rheological properties", POLYMER, ELSEVIER, AMSTERDAM, NL, vol. 45, no. 5, 1 March 2004 (2004-03-01), pages 1569 - 1575, XP004487411, ISSN: 0032-3861, DOI: 10.1016/ J.POLYMER.2003.12.028**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **"Silylated Cellulose in Personal Care Applications", RESEARCH DISCLOSURE, KENNETH MASON PUBLICATIONS, HAMPSHIRE, UK, GB, vol. 681, no. 22, 1 June 2021 (2021-06-01), XP007148993, ISSN: 0374-4353, [retrieved on 20201201]**

## Description

The present invention relates to an oil/polymer blend, a skin care formulation and a cosmetic method.

Consumers increasingly desire skin care formulations that provide long wear properties, such that the formulation might be applied once and last through the work day and beyond without the need for refreshing or touching up. Given todays active lifestyles, it is no simple task to provide such long wear skin care formulations.

An approach to providing such cosmetic formulations is disclosed by Konik et al. in U.S. Patent No. 6,060,072. In U.S. Patent No. 6,060,072, Konik et al. disclose water proof or water resistant cosmetic compositions which comprise a styrene-ethylene-propylene copolymer in an amount of 5 to 10 %, a combination of a PVP/eicosene copolymer and tricontanyl PVP copolymer in an amount of 0.1 to 50 %, a $C_{8-9}$ isoparaffin, a $C_{9-12}$ aliphatic hydrocarbon, or a combination thereof, in an amount of 50 to 85%.

In U.S. Patent Application Publication No. US 2001/0021387, Krammer et al. disclose water-soluble, organosilylated cellulose ethers. The organosilylated cellulose ethers exhibit thickening action in aqueous solution and are suitable as thickeners in paints, adhesives, and cosmetics.

Notwithstanding, there remains a need for new skin care formulations that provide effective wear resistance with color retention.

The present invention provides an oil/polymer blend comprising: (a) 75 to 99.9 wt%, based on the weight of the oil/polymer blend, of an oil, wherein the oil is a cosmetically acceptable oil, selected from the group consisting of linear or branched $C_{8-30}$ alkanes, $C_{8-30}$ alkyl esters, $C_{8-30}$ carboxylic acid esters, $C_{8-30}$ linear or branched alcohols, silicone fluids, and mixtures thereof; (b) 0.1 to 25 wt%, based on the weight of the oil/polymer blend, of a thickening polymer, wherein the thickening polymer is a silylated cellulose polymer, comprising a cellulose polymer functionalized with $-Si(R^1)_3$ groups; wherein each $R^1$ is independently selected from the group consisting of a hydrogen, a $C_{1-8}$ alkyl group, a $C_{1-8}$ haloalkyl group, an aryl group, a $C_{1-8}$ alkylaryl group and a $C_{1-8}$ haloalkylaryl group; wherein the unfunctionalized cellulose polymer has a weight average molecular weight of 50,000 to 1,500,000 Daltons as measured by gel permeation chromatography (GPC).

The present invention also provides a skin care formulation, comprising: a cosmetically acceptable carrier; a color ingredient, wherein the color ingredient is selected from the group consisting of inorganic pigments, organic pigments, dyes, aqueous pigment dispersions and mixtures thereof; and an oil/polymer blend of the present invention.

The present invention also provides a cosmetic method, comprising: providing a skin care formulation of the present invention, and applying the skin care formulation to skin.

## DETAILED DESCRIPTION

We have now surprisingly found the unique oil/polymer blend, as described herein, when formulated into skin care formulations provide effective thickening (preferably, $\geq$ 500 Pa·s at 0.01 $sec^{-1}$ (more preferably, $\geq$ 5,000 Pa·s at 0.01 $sec^{-1}$)); effective shear thinning (preferably, $\leq$ 10 Pa·s at 100 $sec^{-1}$ (more preferably, $\leq$ 0.5 Pa·s at 100 $sec^{-1}$)); and good rub off resistance, which properties benefit an array of skin care compositions including foundation, lip stick, lip gloss, mascara, hair oils, sunscreen oil and antiperspirant/deodorant formulations.

Unless otherwise indicated, ratios, percentages, parts, and the like are by weight.

As used herein, unless otherwise indicated, the terms "weight average molecular weight" and "$M_w$" are used interchangeably to refer to the weight average molecular weight as measured in a conventional manner with gel permeation chromatography (GPC) and conventional standards, such as polystyrene standards. GPC techniques are discussed in detail in Modern Size Exclusion Liquid Chromatography: Practice of Gel Permeation and Gel Filtration Chromatography, Second Edition, Striegel, et al., John Wiley & Sons, 2009. Weight average molecular weights are reported herein in units of Daltons.

The term "cosmetically acceptable" as used herein and in the appended refers to ingredients typically used in personal care compositions, and is intended to underscore that materials that are toxic when present in the amounts typically found in personal care compositions are not contemplated as part of the present invention.

The term "aesthetic characteristics" as used herein and in the appended claims in reference to an acidic aqueous cleansing formulation refers to visual and tactile sensory properties (e.g., smoothness, tack, lubricity, texture, color, clarity, turbidity, uniformity). The oil/polymer blend of the present invention, comprises 75 to 99.9 wt% (preferably, 80 to 99.7 wt%; more preferably, 85 to 99.6 wt%; most preferably, 90 to 99.5 wt%), based on weight of the oil/polymer blend, of an oil, wherein the oil is a cosmetically acceptable oil selected from the group consisting of linear or branched $C_{8-30}$ alkanes (preferably, $C_{14-22}$ mineral oil, isododecane, $C_{11}/C_{13}$ linear alkane blends (e.g., Cetiol Ultimate), hemisqualane; more preferably, isododecane, $C_{11}/C_{13}$ linear alkane blends (e.g., Cetiol Ultimate), most preferably, isododecane); $C_{8-30}$ alkyl esters (preferably, 2-methylundecanoate, $C_{12}$ alkyl benzoate, ethyl decanoate, ethyl dodecanoate, pentaerythrityl tetraethylhexanoate, octyldodecyl neopentanoate, tricaprylin, ethylhexyl isononanoate, methylheptyl isostearate, dicaprylyl maleate, ethylhexyl hydroxystearate); $C_{8-30}$ carboxylic acid esters (preferably, oleic acid, decanoic acid, octanoic

acid, linoleic acid, palmitic acid, stearic acid, lauric acid, myristic acid, ricinoleic acid); $C_{8-30}$ linear or branched alcohols (preferably, 2-butyl-1-octanol, cetyl alcohol (aka 1-hexadecanol), stearyl alcohol, dodecanol (aka lauryl alcohol), octanol (aka capryl alcohol), 1-nonanol, 1-decanol (aka capric alcohol), 1-undecanol, 1-tridecanol, myristyl alcohol (aka 1-tetradecanol), 1-pentadecanol, cis-9-hexadecen-1-ol, heptadecyl alcohol, oleyl alcohol (aka 1-octadecenol), 1-nonadecanol, 1-eicosanol, 1-heneicosanol, behenyl alcohol (aka 1-docosanol), cis-13-docosen-1-ol, 1-tetracosanol, ceryl alcohol (aka 1-hexacosanol), 1-heptacosanol, montanyl alcohol, 1-nonacosanol, myricyl alcohol); silicone fluids (preferably, caprylyl methicone, methylhexyl trisiloxane, phenyl trimethicone, cyclosiloxanes (e.g., cyclopentasiloxane, cyclohexasiloxane), polydimethylsiloxane (e.g., 5 cSt 200 fluid)); and mixtures thereof. Preferably, the oil/polymer blend of the present invention, comprises 75 to 99.9 wt% (preferably, 80 to 99.7 wt%; more preferably, 85 to 99.6 wt%; most preferably, 90 to 99.5 wt%), based on weight of the oil/polymer blend, of an oil, wherein the oil is a cosmetically acceptable oil selected from the group consisting of $C_{14-22}$ mineral oil, isododecane, $C_{11}/C_{13}$ linear alkane blend, hemisqualane, 2-methylundecanoate, $C_{12}$ alkyl benzoate, ethyl decanoate, ethyl dodecanoate, pentaerythrityl tetraethylhexanoate, octyldodecyl neopentanoate, tricaprylin, ethylhexyl isononanoate, methylheptyl isostearate, dicaprylyl maleate, ethylhexyl hydroxystearate, oleic acid, decanoic acid, octanoic acid, linoleic acid, palmitic acid, stearic acid, lauric acid, myristic acid, ricinoleic acid, 2-butyl-1-octanol, cetyl alcohol, stearyl alcohol, dodecanol, octanol, 1-nonanol, 1-decanol, 1-undecanol, 1-tridecanol, myristyl alcohol, 1-pentadecanol, cis-9-hexadecen-1-ol, heptadecyl alcohol, oleyl alcohol, 1-nonadecanol, 1-eicosanol, 1-heneicosanol, behenyl alcohol, cis-13-docosen-1-ol, 1-tetracosanol, ceryl alcohol, 1-heptacosanol, montanyl alcohol, 1-nonacosanol, myricyl alcohol, caprylyl methicone, methylhexyl trisiloxane, phenyl trimethicone, cyclopentasiloxane, cyclohexasiloxane, polydimethylsiloxane and mixtures thereof. Most preferably, the oil/polymer blend of the present invention, comprises 75 to 99.9 wt% (preferably, 80 to 99.7 wt%; more preferably, 85 to 99.6 wt%; most preferably, 90 to 99.5 wt%), based on weight of the oil/polymer blend, of an oil, wherein the oil is a cosmetically acceptable oil selected from the group consisting of isododecane, caprylyl methicone and mixtures thereof.

The oil/polymer blend of the present invention, comprises 0.1 to 25 wt% (preferably, 1 to 20 wt%; more preferably, 2 to 15 wt%; most preferably, 2.5 to 7.5 wt%), based on weight of the oil/polymer blend, of the thickening polymer; wherein the thickening polymer is a silylated cellulose polymer; wherein the silylated cellulose polymer comprises a cellulose polymer functionalized with $-Si(R^1)_3$ groups; wherein the unfunctionalized cellulose polymer has a weight average molecular weight of 50,000 to 1,500,000 Daltons (preferably, 50,000 to 1,250,000 Daltons; more preferably, 80,000 to 1,150,000 Daltons; most preferably, 100,000 to 1,100,000 Daltons) as measured by gel permeation chromatography (GPC); wherein each $R^1$ is independently selected from the group consisting of a hydrogen, a $C_{1-8}$ alkyl group, a $C_{1-8}$ haloalkyl group, an aryl group, a $C_{1-8}$ alkylaryl group and a $C_{1-8}$ haloalkylaryl group. Preferably, the oil/polymer blend of the present invention, comprises 0.1 to 25 wt% (preferably, 1 to 20 wt%; more preferably, 2 to 15 wt%; most preferably, 2.5 to 7.5 wt%), based on weight of the oil/polymer blend, of a thickening polymer, wherein the thickening polymer is a silylated cellulose polymer; wherein the silylated cellulose polymer comprises a cellulose polymer functionalized with $-Si(R^1)_3$ groups; wherein the unfunctionalized cellulose polymer has a weight average molecular weight of 50,000 to 1,500,000 Daltons (preferably, 50,000 to 1,250,000 Daltons; more preferably, 80,000 to 1,150,000 Daltons; most preferably, 100,000 to 1,100,000 Daltons) as measured by gel permeation chromatography (GPC); wherein each $R^1$ is independently selected from the group consisting of a hydrogen and a $C_{1-8}$ alkyl group. More preferably, the oil/polymer blend of the present invention, comprises 0.1 to 25 wt% (preferably, 1 to 20 wt%; more preferably, 2 to 15 wt%; most preferably, 2.5 to 7.5 wt%), based on weight of the oil/polymer blend, of a thickening polymer, wherein the thickening polymer is a silylated cellulose polymer; wherein the silylated cellulose polymer comprises a cellulose polymer functionalized with $-Si(R^1)_3$ groups; wherein the unfunctionalized cellulose polymer has a weight average molecular weight of 50,000 to 1,500,000 Daltons (preferably, 50,000 to 1,250,000 Daltons; more preferably, 80,000 to 1,150,000 Daltons; most preferably, 100,000 to 1,100,000 Daltons) as measured by gel permeation chromatography (GPC); wherein each $R^1$ is independently selected from the group consisting of a $C_{1-4}$ alkyl group. Still more preferably, the oil/polymer blend of the present invention, comprises 0.1 to 25 wt% (preferably, 1 to 20 wt%; more preferably, 2 to 15 wt%; most preferably, 2.5 to 7.5 wt%), based on weight of the oil/polymer blend, of a thickening polymer, wherein the thickening polymer is a silylated cellulose polymer; wherein the silylated cellulose polymer comprises a cellulose polymer functionalized with $-Si(R^1)_3$ groups; wherein the unfunctionalized cellulose polymer has a weight average molecular weight of 50,000 to 1,500,000 Daltons (preferably, 50,000 to 1,250,000 Daltons; more preferably, 80,000 to 1,150,000 Daltons; most preferably, 100,000 to 1,100,000 Daltons) as measured by gel permeation chromatography (GPC); wherein each $R^1$ is independently selected from the group consisting of a $C_{1-2}$ alkyl group. Most preferably, the oil/polymer blend of the present invention, comprises 0.1 to 25 wt% (preferably, 1 to 20 wt%; more preferably, 2 to 15 wt%; most preferably, 2.5 to 7.5 wt%), based on weight of the oil/polymer blend, of a thickening polymer, wherein the thickening polymer is a silylated cellulose polymer; wherein the silylated cellulose polymer comprises a cellulose polymer functionalized with $-Si(R^1)_3$ groups; wherein the unfunctionalized cellulose polymer has a weight average molecular weight of 50,000 to 1,500,000 Daltons (preferably, 50,000 to 1,250,000 Daltons; more preferably, 80,000 to 1,150,000 Daltons; most preferably, 100,000 to 1,100,000 Daltons) as measured by gel permeation chromatography (GPC); wherein each $R^1$ is a methyl group.

Preferably, the oil/polymer blend of the present invention, comprises 0.1 to 25 wt% (preferably, 1 to 20 wt%; more

preferably, 2 to 15 wt%; most preferably, 2.5 to 7.5 wt%), based on weight of the oil/polymer blend, of a thickening polymer, wherein the thickening polymer is a silylated cellulose polymer of formula I

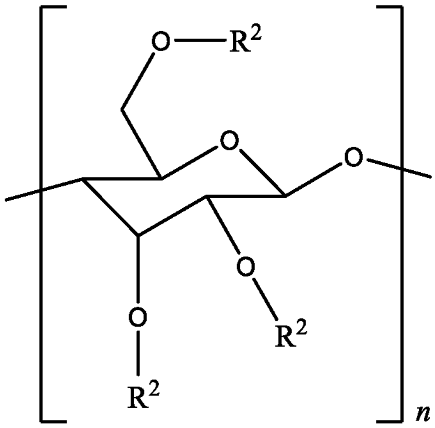

wherein *n* is an average of 308 to 9,252 (preferably, 308 to 6,167; more preferably, 3083 to 4,317); wherein each $R^2$ is independently selected from the group consisting of a hydrogen, a $C_{1-8}$ alkyl group and a $-Si(R^1)_3$ group (preferably, a hydrogen, a $C_{1-4}$ alkyl group and a $-Si(R^1)_3$ group; more preferably, a hydrogen, a $C_{1-2}$ alkyl group and a $-Si(R^1)_3$ group; most preferably, a hydrogen and a $-Si(R^1)_3$ group); wherein each $R^1$ is independently selected from the group consisting of a hydrogen, a $C_{1-8}$ alkyl group, a $C_{1-8}$ haloalkyl group, an aryl group, a $C_{1-8}$ alkylaryl group and a $C_{1-8}$ haloalkylaryl group (preferably, a hydrogen and a $C_{1-8}$ alkyl group; more preferably, a $C_{1-4}$ alkyl group; still more preferably, a $C_{1-2}$ alkyl group; most preferably, a methyl group).

Preferably, the thickening polymer is a silylated cellulose polymer; wherein the silylated cellulose polymer comprises a cellulose polymer functionalized with $-Si(R^1)_3$ groups having a degree of substitution, DS, of the $-Si(R^1)_3$ groups of 1.4 to 3.0 (preferably, 1.5 to 3.0; more preferably, 2.0 to 3.0; most preferably, 2.25 to 3.0) determined by Attenuated Total Reflection - Fourier Transform Infrared (ATR-FTIR) Spectroscopy.

The skin care formulation of the present invention comprises: a cosmetically acceptable carrier (preferably, 30 to 92 wt% (more preferably, 35 to 92 wt%; most preferably, 40 to 80 wt%), based on weight of the skin care formulation, of the cosmetically acceptable carrier); a color ingredient (preferably, 0.01 to 25 wt% (more preferably, 0.1 to 20 wt%; most preferably, 1 to 10 wt%), based on weight of the skin care formulation, of the color ingredient); and an oil/polymer blend of the present invention (preferably, 5 to 69.9 wt% (more preferably, 7 to 50 wt%; most preferably, 15 to 30 wt%), based on weight of the skin care formulation, of the oil/polymer blend). Preferably, the skin care formulation of the present invention, comprises 30 to 92 wt% (preferably, 35 to 92 wt%; more preferably, 40 to 80 wt%), based on weight of the skin care formulation, of a cosmetically acceptable carrier. More preferably, the skin care formulation of the present invention, comprises 30 to 92 wt% (preferably, 35 to 92 wt%; more preferably, 40 to 80 wt%), based on weight of the skin care formulation, of a cosmetically acceptable carrier; wherein the color ingredient and the oil/polymer blend are dispersed in the cosmetically acceptable carrier. Still more preferably, the skin care formulation of the present invention, comprises 30 to 92 wt% (preferably, 35 to 92 wt%; more preferably, 40 to 80 wt%), based on weight of the skin care formulation, of a cosmetically acceptable carrier; wherein the color ingredient and the oil/polymer blend are dispersed in the cosmetically acceptable carrier. Most preferably, the skin care formulation of the present invention, comprises 30 to 92 wt% (preferably, 35 to 92 wt%; more preferably, 40 to 80 wt%), based on weight of the skin care formulation, of a cosmetically acceptable carrier; wherein the color ingredient and the oil/polymer blend are dispersed in the cosmetically acceptable carrier.

Preferably, the skin care formulation of the present invention, comprises 30 to 92 wt% (preferably, 35 to 92 wt%; more preferably, 40 to 80 wt%), based on weight of the skin care formulation, of a cosmetically acceptable carrier; wherein the cosmetically acceptable carrier is selected to be capable of evaporating upon application of the skin care formulation to mammalian skin (preferably, human skin).

Preferably, the skin care formulation of the present invention, comprises 30 to 92 wt% (preferably, 35 to 92 wt%; more preferably, 40 to 80 wt%), based on weight of the skin care formulation, of a cosmetically acceptable carrier; wherein the cosmetically acceptable is selected from the group consisting of water (e.g., deionized, distilled water); short chain alcohols (e.g., $C_{1-4}$ straight or branched chain alcohols such as methyl alcohol, ethyl alcohol, propyl alcohol, isopropyl alcohol, butyl alcohol); glycols (e.g., ethylene glycol, propylene glycol, butylene glycol, pentylene glycol, hexylene glycol, dipropylene glycol, ethoxydiglycol); cyclosiloxanes (e.g., cyclopentasiloxane, cyclohexasiloxane); glycerin; isododecane; isohexadecane; acetone; methyl acetate; butyl cellosolve and mixtures thereof. More preferably, the skin care formulation of the present invention, comprises 30 to 92 wt% (preferably, 35 to 92 wt%; more preferably, 40 to 80 wt%), based on weight of the skin care formulation, of a cosmetically acceptable carrier; wherein the cosmetically acceptable carrier includes at

least one of water and isododecane (preferably, at least one of deionized water, distilled water and isododecane; more preferably, at lease one of deionized water and isododecane).

The skin care formulation of the present invention comprises a color ingredient; wherein the color ingredient is selected from the group consisting of inorganic pigments, organic pigments, dyes, aqueous pigment dispersions and mixtures thereof. Preferably, the skin care formulation of the present invention comprises a color ingredient; wherein the color ingredient is selected from the group consisting of Ext. D&C Yellow No. 2, Ext. D & C Violet No. 2, FD&C Red No. 4, FD&C Red No. 40, FD&C Yellow No. 5, FD&C Yellow No. 6, FD&C Green No. 3, FD&C Blue No. 1, D&C Yellow No. 7, D&C Yellow No. 8, D&C Yellow No. 10, D&C Yellow No. 11, D&C Violet No. 2, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 34, D&C Red No. 33, D&C Red No. 36, D&C Green No. 5, D&C Green No. 6, D&C Green No. 8, D&C Blue No. 4, D&C Orange No. 4, D&C Orange No. 5, D&C Orange No. 10, D&C Orange No. 11, D&C Brown No. 1, Aluminum powder, Annatto, Bismuth citrate, Bismuth Oxychloride, Bronze powder, Caramel, Carmine, β-Carotene, Chromium hydroxide green, Chromium oxide green, Copper chlorophyllin, Copper powder, Dihydroxyacetone, Ferric Ammonium ferrocyanide, Ferric ferrocyanide, Guanine, Iron oxide, Manganese Violet, Mica, Silver, Titanium Dioxide, Ultramarine, Zinc Oxide and mixtures thereof. More preferably, the skin care formulation of the present invention comprises a color ingredient; wherein the color ingredient includes at least one iron oxide and titanium dioxide. Most preferably, preferably, the skin care formulation of the present invention comprises a color ingredient; wherein the color ingredient includes a mixture of iron oxides and titanium dioxide.

Preferably, the skin care formulation of the present invention, optionally, further comprises an optional additive. More preferably, the skin care formulation of the present invention, further comprises an optional additive, wherein the optional additive is selected from the group consisting of suncare actives, film formers, emollients, preservatives, antioxidants, fragrances, humectants, rheology modifiers, aesthetic modifiers, vitamins, skin protectants, oils, emulsifiers, surfactants, pearlizers, consistency factors, thickeners, super fatting agents, stabilizers, polymers, silicone compounds, fats, waxes, lecithins, phospholipids, fillers, light management powders and particles, and mixtures thereof.

Preferably, the skin care formulation of the present invention, further comprises a suncare active. More preferably, the skin care formulation of the present invention further comprises a suncare active; wherein the suncare active is a UV radiation absorbing agent. Still more preferably, the skin care formulation of the present invention, further comprises a suncare active, wherein the suncare active is a UV radiation absorbing agent selected from the group consisting of physical blockers (e.g., red petrolatum, titanium dioxide, zinc oxide) and chemical absorbers (e.g., 1-(4-methoxyphenol)-3-(4-tert-butylphenyl)propane-1,3-dione (INCI: Butyl Methoxydibenzoylmethane); 2-hydroxy-4-methoxybenzophenone (INCI: Benzophenone-3); dioxybenzone; sulisobenzone; menthyl anthranilate; para-aminobenzoic acid; amyl paradimethylaminobenzoic acid; octyl para-dimethylaminobenzoate; ethyl 4-bis (hydroxypropyl) para-aminobenzoate; polyethylene glycol (PEG-25) para-aminobenzoate; ethyl 4-bis (hydroxypropyl) aminobenzoate; diethanolamine para-methyoxycinnamate; 2-ethoxyethyl para-methoxycinnamate; ethylhexyl para-methoxycinnamate; octyl para-methoxycinnamate; isoamyl para-methoxycinnamate; 2-ethylhexyl-2-cyano-3,3-diphenyl-acrylate; 2-ethylhexyl-2-cyano-3,3-diphenyl-2-propenoate (INCI: octocrylene); 2-ethylhexyl-2-hydroxybenzoate (INCI: Ethylhexyl Salicylate); homomenthyl salicylate; glyceryl aminobenzoate; triethanolamine salicylate; digalloyl trioleate; lawsone with dihydroxyacetone; 2-phenylbenzimidazole-5-sulfonic acid; 4-methylbenzylidine camphor; avobenzone; triazines; benzotriazoles; vinyl group-containing amides; cinnamic acid amides; sulfonated benzimidazoles); 3,3,5-trimethylcyclohexyl 2-hydroxybenzoate (INCI: Homosalate). Yet more preferably, the skin care formulation of the present invention, further comprises of a suncare active, wherein the suncare active is a UV radiation absorbing agent comprises a mixture of UV radiation absorbing agents. Most preferably, the skin care formulation of the present invention, comprises a suncare active, wherein the suncare active is a UV radiation absorbing agent is a mixture of UV absorbing agents including at least one of titanium dioxide; 1-(4-methoxyphenol)-3-(4-tert-butylphenyl)propane-1,3-dione; 2-ethylhexyl-2-hydroxybenzoate; 2-ethyhexyl-2-cyano-3,3-diphenyl-2-propenoate; 2-hydroxy-4-methoxybenzophenone and 3,3,5-trimethylcyclohexyl 2-hydroxybenzoate.

Preferably, the skin care formulation of the present invention has a pH of 4 to 9. More preferably, the skin care formulation of the present invention has a pH of 4.5 to 8.5. Still more preferably, the skin care formulation of the present invention has a pH of 5.0 to 8.0. Most preferably, the skin care formulation of the present invention has a pH of 5.5 to 7.5.

Preferably, the skin care formulation of the present invention is provided a product form selected from the group consisting of a cream, an aqueous solution, an emulsion (e.g., water-in-oil emulsion, oil-in-water emulsion), an oil, an ointment, a paste, a gel, a lotion, a milk, a foam, a stick and a suspension.

The skin care formulation of the present invention are useful for enhancing the appearance of skin through application to the skin. Preferably, the skin care formulation of the present invention applies easily to the skin, leaving a clear vivid color that remains in place at least through the work day and preferably thereafter.

Some embodiments of the present invention will now be described in detail in the following **Examples.**

**Examples S1-S25: Synthesis of Thickening Polymer**

To a two liter three-neck flask equipped with a nitrogen inlet and a temperature controller was weighed the type and amount of cellulose noted in **TABLE 1.** Then N,N-dimethylacetamide (DMAc) solvent was added to the flask in the amount noted in **TABLE 1.** The flask contents were then placed under an atmosphere of nitrogen. Then hexamethyldisilazane (HMDS) was added to the flask contents all at once in the amount noted in **TABLE 1.** The flask contents were then slowly heated while stirring to the set temperature, $T_1$, noted in **TABLE 1** and stirred for time, $t^1$, in hours noted in **TABLE 1.** The heat source was then removed and the flask contents were left to cool. Then xylene (Xyl) was added to the flask contents in the amount noted in **TABLE 1** and the flask contents were then heated to set temperature, $T_2$, noted in **TABLE 1** and stirred for time, $t^2$, in hours noted in **TABLE 1.** The product solution was then transferred to a separatory funnel and subjected to non-solvent precipitation by dropwise addition into two liters of vigorously stirring methanol. The product was precipitated in the form of a thread or flakes. The product was isolated by filtration, and the product was dried in a vacuum oven at 50 °C overnight. The product was then suspended in 500 mL of methanol and left overnight, then refiltered and dried in vacuum oven at 50 °C overnight.

**TABLE 1**

| Example | Cellu lose | | DMAc (g) | HMDS(g) | $T_1$ (°C) | $t^1$ (hr) | Xyl (g) | $T_2$ (°C) | $t^2$ (hr) |
|---|---|---|---|---|---|---|---|---|---|
| | Type | (g) | | | | | | | |
| S1 | A | 15.2 | 300 | 40.0 | 140 | 3.0 | 400 | 140 | 4.0 |
| S2 | B | 15.2 | 330 | 50.0 | 140 | 2.0 | 320 | 140 | 4.0 |
| S3 | B | 15.2 | 400 | 50.0 | 140 | 2.0 | 600 | 140 | 4.0 |
| S4 | A | 15.0 | 300 | 50.0 | 130 | 5.0 | 300 | 140 | 6.0 |
| S5 | C | 15.1 | 330 | 49.9 | 130 | 7.5 | 400 | 125 | 5.0 |
| S6 | D | 15.0 | 331 | 49.8 | 130 | 7.5 | 400 | 125 | 5.0 |
| S7 | E | 15.0 | 330 | 50.0 | 130 | 5.0 | 400 | 140 | 6.0 |
| S8 | C | 15.2 | 333 | 149.9 | 135 | 3.5 | 400 | 135 | 5.0 |
| S9 | D | 15.1 | 332 | 150.1 | 135 | 3.5 | 400 | 135 | 5.0 |
| S10 | F | 15.5 | 329 | 49.7 | 135 | 5.0 | 400 | 120 | 3.0 |
| S11 | G | 15.4 | 329 | 50.0 | 135 | 5.0 | 400 | 120 | 3.0 |
| S12 | C | 2.0 | 39 | 5.96 | 130 | 4.0 | 0 | -- | -- |
| S13 | H | 15.2 | 337 | 50.1 | 135 | 6.0 | 400 | 135 | 3.5 |
| S14 | G | 15.2 | 315 | 49.7 | 135 | 4.0 | 400 | 130 | 3.0 |
| S15 | G | 15.2 | 308 | 50.2 | 135 | 4.0 | 400 | 130 | 3.0 |
| S16 | G | 15.2 | 315 | 98.7 | 80 | 4.0 | 300 | 130 | 5.5 |
| S17 | G | 15.3 | 308 | 99.0 | 80 | 4.0 | 320 | 130 | 5.5 |
| S18 | F | 15.1 | 312 | 50.0 | 130 | 5.0 | 400 | 105 | 8.0 |
| S19 | G | 15.2 | 324 | 50.2 | 130 | 5.0 | 400 | 105 | 8.0 |
| S20 | E | 15.2 | 318 | 49.9 | 130 | 4.0 | 330 | 130 | 5.0 |
| S21 | C | 15.0 | 328 | 49.6 | 130 | 4.0 | 330 | 130 | 5.0 |
| S22 | G | 15.2 | 329 | 19.9 | 130 | 4.0 | 330 | 120 | 2.0 |
| S23 | G | 15.2 | 326 | 30.0 | 130 | 4.0 | 330 | 120 | 2.0 |
| S24 | A | 15.2 | 327 | 49.9 | 130 | 4.0 | 330 | 130 | 4.0 |
| S25 | A | 15.2 | 378 | 49.9 | 130 | 4.0 | 330 | 130 | 4.0 |

[A] Avicel PH-101 (51 μm) microcrystalline cellulose having weight average molecular weight of 60,000 Daltons available from Sigma-Aldrich

(continued)

| Example | Cellu lose Type | Cellu lose (g) | DMAc (g) | HMDS(g) | $T_1$ (°C) | $t^1$ (hr) | Xyl (g) | $T_2$ (°C) | $t^2$ (hr) |
|---|---|---|---|---|---|---|---|---|---|
| [B] Crystal PL HD eucalyptus pulp having weight average molecular weight of 400,000 Daltons available from Bahi Specialty Cellulose | | | | | | | | | |
| [C] Biofloc 92 MV wood pulp having weight average molecular weight of 600,000 Daltons available from Tartas (Rayonier) | | | | | | | | | |
| [D] Biofloc XV wood pulp having weight average molecular weight of 900,000 Daltons available from Tartas (Rayonier) | | | | | | | | | |
| [E] PCS-2400 cotton linters having weight average molecular weight of 1,000,000 Daltons available from Gaomi | | | | | | | | | |
| [F] E4 wood pulp having weight average molecular weight of 100,000 Daltons available from GP Cellulose | | | | | | | | | |
| [G] E60-HB wood pulp having weight average molecular weight of 125,000 Daltons available from GP Cellulose | | | | | | | | | |
| [H] Biofloc 94 MV wood pulp having weight average molecular weight of 600,000 Daltons available from Tartas (Rayonier) | | | | | | | | | |

**DS Determination**

The degree of substitution, DS, of $-Si(CH_3)_3$ in the thickening polymers prepared according to **Examples S1-S25** was determined using well known techniques based on Attenuated Total Reflection - Fourier Transform Infrared Spectroscopy analyzing spectra peak areas calculated with MATLAB using the spectral parameters provided in **TABLE 2** with the DS values determined as reported in **TABLE 3.**

**TABLE 2**

| Species | Integration ($cm^{-1}$) | | Baseline ($cm^{-1}$) | |
|---|---|---|---|---|
| | Start | End | Start | End |
| $-Si(CH_3)_3$ | 1277 | 1220 | 1278 | 1219 |
| -OH | 3685 | 3033 | 3697 | 3040 |

**TABLE 3**

| Example | IR Peak Area | | | DS | Si (wt%) | OH (wt%) |
|---|---|---|---|---|---|---|
| | $Si(CH_3)_3$ | CO | OH | | | |
| S2 | 1.28 | 9.92 | 0.264 | 2.58 | 20.8 | 2.04 |
| S3 | 1.47 | 12.0 | 0.686 | 2.20 | 19.2 | 4.26 |
| S4 | 1.44 | 13.3 | 1.59 | 1.61 | 16.2 | 8.49 |
| S5 | 0.963 | 7.35 | 0.426 | 2.23 | 19.4 | 4.07 |
| S6 | 1.62 | 12.8 | 0.714 | 2.23 | 19.4 | 4.06 |
| S7 | 0.597 | 4.95 | 0.351 | 2.05 | 18.6 | 5.19 |
| S8 | 0.938 | 7.24 | 0.282 | 2.43 | 20.2 | 2.89 |
| S9 | 0.760 | 6.51 | 0.382 | 2.15 | 19.0 | 4.54 |
| S10 | 1.08 | 8.49 | 0.248 | 2.54 | 20.6 | 2.26 |
| S11 | 1.03 | 7.67 | 0.0194 | 2.96 | 22.1 | 0.20 |
| S12 | 1.13 | 7.84 | -0.0337 | 3.00 | 22.2 | 0 |
| S13 | 0.811 | 8.81 | 1.51 | 1.22 | 13.7 | 12.1 |
| S14 | 1.63 | 12.6 | 0.237 | 2.69 | 21.2 | 1.47 |
| S15 | 2.12 | 15.3 | -0.151 | 3.00 | 22.2 | 0 |
| S16 | 1.34 | 9.58 | -0.00681 | 3.00 | 22.2 | 0 |

(continued)

| Example | IR Peak Area | | | DS | Si (wt%) | OH (wt%) |
|---|---|---|---|---|---|---|
| | $Si(CH_3)_3$ | CO | OH | | | |
| **S17** | 0.623 | 4.47 | 0.0535 | 2.81 | 21.6 | 0.88 |
| **S18** | 0.933 | 6.84 | 0.102 | 2.76 | 21.4 | 1.11 |
| **S19** | 1.08 | 7.82 | 0.215 | 2.60 | 20.8 | 1.97 |
| **S20** | 1.33 | 9.27 | -0.0648 | 3.00 | 22.2 | 0 |
| **S21** | 0.491 | 3.48 | -0.00282 | 3.00 | 22.2 | 0 |
| **S22** | 0.205 | 2.09 | 0.165 | 1.84 | 17.5 | 6.70 |
| **S23** | 1.11 | 8.44 | 0.248 | 2.55 | 20.7 | 2.20 |
| **S24** | 0.728 | 10.5 | 2.92 | 0.72 | 9.47 | 18.1 |
| **S25** | 2.02 | 15.1 | -0.218 | 3.00 | 22.2 | 0 |

**Comparative Example C1 and Examples 1-4: Skin Care Formulations**

The skin care formulations of **Comparative Example C1** and **Examples 1-4** were prepared having the formulation noted in **TABLE** 4. The Phase B ingredients were mixed in a separate container. The Phase C ingredients were combined in a separate container with mixing until uniform. The Phase A and D ingredients were combined in a separate beaker and mixed until uniform. The mixed Phase C ingredients were then added to the combined Phase A and D ingredients with shear at 450 rpm until homogeneous. Then the mixed Phase B ingredients were then slowly added to the combined Phase A, C and D ingredients with shear at 700 rpm. The combined formulation was then mixed for an additional 5 minutes with shear at 2,000 rpm to provide the product skin care formulation.

**TABLE 4**

| Phase | Ingredient INCI name | Example | | | | |
|---|---|---|---|---|---|---|
| | | C1 | 1 | 2 | 3 | 4 |
| | | (wt%) | (wt%) | (wt%) | (wt%) | (wt%) |
| A | Lauryl PEG-10 Tris(trimethylsiloxy)silyethyl Dimeticone[1] | 6 | 6 | 6 | 6 | 6 |
| A | Caprylyl methicone[2] | 4 | 4 | 4 | 4 | 4 |
| A | Isododecane[3] | 15.75 | 18 | 18 | 18 | 18 |
| B | Deionized water | 53 | 53 | 53 | 53 | 53 |
| B | Sodium chloride | 1 | 1 | 1 | 1 | 1 |
| B | Phenoxyethanol (and) Ethylhexylglycerin[4] | 1 | 1 | 1 | 1 | 1 |
| B | Glycerin | 5 | 5 | 5 | 5 | 5 |
| D | Iron Oxide (CI 77499), dimethicone[5] | 0.07 | 0.07 | 0.07 | 0.07 | 0.07 |
| C | Iron Oxide (CI 77491), dimethicone[6] | 0.25 | 0.25 | 0.25 | 0.25 | 0.25 |
| C | Iron Oxide (CI 77492), dimethicone[7] | 1.09 | 1.09 | 1.09 | 1.09 | 1.09 |
| C | Titanium Dioxide (and) dimethicone[8] | 5.81 | 5.81 | 5.81 | 5.81 | 5.81 |
| C | Caprylyl Methicone[2] | 3.28 | 3.28 | 3.28 | 3.28 | 3.28 |
| D | Isododecane (and) Acrylates / Polytimethylsiloxymethacrylate Copolymer[9] | 3.75 | -- | -- | -- | -- |
| D | **Example S16** | -- | 1.5 | -- | -- | -- |
| D | **Example S17** | -- | -- | 1.5 | -- | -- |
| D | **Example S20** | -- | -- | -- | 1.5 | -- |

(continued)

| Phase | Ingredient INCI name | Example | | | | |
|---|---|---|---|---|---|---|
| | | C1 | 1 | 2 | 3 | 4 |
| | | (wt%) | (wt%) | (wt%) | (wt%) | (wt%) |
| D | **Example S21** | -- | -- | -- | -- | 1.5 |

[1] Available from The Dow Chemical Company under tradename DOWSIL™ ES-5300.
[2] Available from The Dow Chemical Company under tradename DOWSIL™ FZ-3196.
[3] Available from The Innovation Company under tradename Creasil ID CG.
[4] Available from Schulke Inc. under tradename Euxyl PE 9010.
[5] Available from Miyoshi America under tradename SAT-B-335198.
[6] Available from Miyoshi America under tradename SAT-Y-338075.
[7] Available from Miyoshi America under tradename SAT-R-338073.
[8] Available from Miyoshi America under tradename SAT-TRI-77891.
[9] Available from The Dow Chemical Company under tradename DOWSIL™ FA4002 ID.

## Thickening and shear thinning

The viscosity of the skin care formulations of **Comparative Example C1** and **Examples 1-4** was determined using a TA Instruments DHR-3 rheometer at 25 °C, equipped with a stainless steel 60 mm, 0.5° cone and plate sensor, a gap set at 17 microns and a shear rate of 6.31 $s^{-1}$. Shear sweep method was used with shear rate ranges from 0.01 $s^{-1}$ to 100 $s^{-1}$. Viscosities measured at low shear (0.1 $s^{-1}$) and high shear (100 $s^{-1}$) are reported in **TABLE 5.**

The shear thinning efficiency of the thickeners was assessed by fitting the viscosity data to the power-law fluid equation

$$\eta = K \cdot \gamma^{n-1}$$

wherein $\eta$ is the viscosity, $\gamma$ is the shear rate, K is the flow consistency index and *n* is the flow behavior index. For a shear thinning fluid, $n < 1$. Smaller *n* values indicate a higher degree of shear thinning. The calculated shear thinning efficiencies for the thickeners is reported in **TABLE 5.**

As shown in **TABLE 5,** data for all of the thickeners fitted well to the power-law fluid equation, with the coefficient of determination, $r^2$, value being close to 1. The flow behavior index (*n*) for the inventive thickeners was much lower than the commercial material (DOWSIL™ FA4002 ID Silicone Acrylate), indicating a much higher degree of sheer thinning which is highly favorable in personal care formulations for better sprayability and spreadability of the formulation. The inventive polymer also lead to a much higher increase in formulation viscosity than the commercial material (DOWSIL™ FA4002 ID Silicone Acrylate), indicating a much higher thickening efficiency for the inventive polymer.

**TABLE 5**

| Skin Care Formulation | Thickener | Viscosity (Pa·s) | | Fitted power law equation | Flow behavior index, *n* |
|---|---|---|---|---|---|
| | | 0.01 $sec^{-1}$ | 100 $sec^{-1}$ | | |
| **C1** | Commercial material[1] | 3.99 | 0.4 | $\eta=1.125\cdot\gamma^{-0.256}$ $r^2 = 0.967$ | 0.744 |
| **1** | **Example S16** | 795 | 2.5 | $\eta=36.351\cdot\gamma^{-0\ 064}$ $r^2 = 0.9947$ | 0.360 |
| **2** | Example **S17** | 1,333 | 2.8 | $\eta=45.241\cdot\gamma^{-0.683}$ $r^2 = 0.9901$ | 0.317 |
| **3** | **Example S20** | 3,276 | 3.9 | $\eta=79.197\cdot\gamma^{-0\ 736}$ $r^2 = 0.9921$ | 0.264 |

(continued)

| Skin Care Formulation | Thickener | Viscosity (Pa·s) | | Fitted power law equation | Flow behavior index, *n* |
|---|---|---|---|---|---|
| | | 0.01 $sec^{-1}$ | 100 $sec^{-1}$ | | |
| 4 | Example S21 | 2,520 | 2.6 | $\eta=57.845\cdot\gamma^{-0.767}$ $r^2 = 0.993$ | 0.233 |
| [1] DOWSIL™ FA4002 ID Silicon Isododecane (and) Acrylates / Polytrimethylsiloxymethacrylate Copolymer available from The Dow Chemical Company | | | | | |

**Comparative Example C2-C3 and Examples 5-9: Skin Care Formulations**

The skin care formulations of **Comparative Examples C2-C3** and **Examples 5-9** were prepared having the formulation noted in **TABLE 6.** The Phase B ingredients were mixed in a separate container. The Phase C ingredients were combined in a separate container with mixing until uniform. The Phase A and D ingredients were combined in a separate beaker and mixed until uniform. The mixed Phase C ingredients were then added to the combined Phase A and D ingredients with shear at 450 rpm until homogeneous. Then the mixed Phase B ingredients were then slowly added to the combined Phase A, C and D ingredients with shear at 700 rpm. The combined formulation was then mixed for an additional 5 minutes with shear at 2,000 rpm to provide the product skin care formulation.

**TABLE 6**

| Phase | Ingredient INCI name | Example (wt %) | | | | | | |
|---|---|---|---|---|---|---|---|---|
| | | C2 | C3 | 5 | 6 | 7 | 8 | 9 |
| A | Lauryl PEG-10 Tris(trimethylsiloxy)silyethyl Dimeticone[1] | 6 | 6 | 6 | 6 | 6 | 6 | 6 |
| A | Caprylyl methicone[2] | 3.6 | 3 | 3 | 3 | 3 | 3 | 3 |
| A | Isododecane[3] | 12 | 11.1 | 12 | 12 | 12 | 12 | 12 |
| B | Deionized water | 60.9 | 60.9 | 60.9 | 60.9 | 60.9 | 60.9 | 60.9 |
| B | Sodium chloride | 1 | 1 | 1 | 1 | 1 | 1 | 1 |
| B | Phenoxyethanol (and) Ethylhexylglycerin[4] | 1 | 1 | 1 | 1 | 1 | 1 | 1 |
| B | Glycerin | 5 | 5 | 5 | 5 | 5 | 5 | 5 |
| D | Iron Oxide (CI 77499), dimethicone[5] | 0.07 | 0.07 | 0.07 | 0.07 | 0.07 | 0.07 | 0.07 |
| C | Iron Oxide (CI 77491), dimethicone[6] | 0.25 | 0.25 | 0.25 | 0.25 | 0.25 | 0.25 | 0.25 |
| C | Iron Oxide (CI 77492), dimethicone[7] | 1.09 | 1.09 | 1.09 | 1.09 | 1.09 | 1.09 | 1.09 |
| C | Titanium Dioxide (and) dimethicone[8] | 5.81 | 5.81 | 5.81 | 5.81 | 5.81 | 5.81 | 5.81 |
| C | Caprylyl Methicone[2] | 3.28 | 3.28 | 3.28 | 3.28 | 3.28 | 3.28 | 3.28 |
| D | Isododecane (and) Acrylates / Polytimethyl-siloxymethacryla te Copolymer[9] | -- | 1.5 | -- | -- | -- | -- | -- |
| D | **Example S8** | -- | -- | 0.6 | -- | -- | -- | -- |
| D | **Example S14** | -- | -- | -- | 0.6 | -- | -- | -- |
| D | **Example S15** | -- | -- | -- | -- | 0.6 | - | -- |
| D | **Example S16** | -- | -- | -- | -- | -- | 0.6 | -- |
| D | **Example S17** | -- | -- | -- | -- | -- | -- | 0.6 |

[1] Available from The Dow Chemical Company under tradename DOWSIL™ ES-5300.
[2] Available from The Dow Chemical Company under tradename DOWSIL™ FZ-3196.
[3] Available from The Innovation Company under tradename Creasil ID CG.
[4] Available from Schulke Inc. under tradename Euxyl PE 9010.
[5] Available from Miyoshi America under tradename SAT-B-335198.

(continued)

| Phase | Ingredient INCI name | Example (wt %) | | | | | | |
|---|---|---|---|---|---|---|---|---|
| | | C2 | C3 | 5 | 6 | 7 | 8 | 9 |

[6] Available from Miyoshi America under tradename SAT-Y-338075.
[7] Available from Miyoshi America under tradename SAT-R-338073.
[8] Available from Miyoshi America under tradename SAT-TRI-77891.
[9] Available from The Dow Chemical Company under tradename DOWSIL™ FA4002 ID.

## Wear Resistance

The skin care formulations prepared according to **Comparative Examples C2-C3** and **Examples 5-9** were each coated on a white vinyl chart (available from Leneta) using a doctor blade film applicator with the gap set at 6 mils (0.1524 mm) and allowed to dry at 22 °C for at least 48 hours. The color reading of each sample was then measured by colorimeter (Ocean Optics). The wear resistance of the deposited film of skin care formulations was characterized by the change (ΔE) before and after abrasion with a pre-cut bath towel (55 mm x 45 mm). The bath towel was fixed to a moving robotic part that moves back and forth periodically at a constant speed. The film was abraded by the bath towel by 3 and 6 wear cycles under a pressure of approximately 600 Pa, each wear cycle lasts 6 seconds. Readings were taken from ten point on each deposited film. The average of the readings from each sample is provided in **TABLE 7.** Lower ΔE indicates less color change after rub-off and higher wear resistance. The ΔE of the inventive polymers were lower than the commercial material, which is highly favorable for skin care formulations to enable long lasting performance.

**TABLE 7**

| Skin care formulation | Wear Cycles | |
|---|---|---|
| | 3 | 6 |
| | ΔE | ΔE |
| **Comp. Example C2** | 10.51 | 12.03 |
| **Comp. Example C3** | 7.86 | 11.44 |
| **Example 5** | 5.70 | 7.85 |
| **Example 6** | 4.96 | 7.40 |
| **Example 7** | 8.07 | 10.93 |
| **Example 8** | 5.60 | 8.62 |
| **Example 9** | 7.15 | 8.47 |

## Comparative Examples C4-C13 and Examples 10-28: Viscosity

The viscosity of oils alone, **Comparative Examples C4-C13,** and thickening oil/polymer blends, **Examples 10-28,** as noted in **TABLE 8** was determined using a TA Instruments DHR-3 rheometer at 25 °C, equipped with a stainless steel 60 mm, 0.5° cone and plate sensor, a gap set at 17 microns and a shear rate of 0.01 $s^{-1}$. The viscosities measured are reported in **TABLE 8.**

**TABLE 8**

| Example | Oil | Thickening Polymer ("TP") | Conc. TP in Oil (wt%) | Viscosity (mPa·s) |
|---|---|---|---|---|
| **C4** | XIAMETER™ PMX-200 Silicone Fluid, 0.65 cSt[a] | -- | -- | $4.81 \times 10^{-1}$ |
| **C5** | Cetiol Ultimate[b] | -- | -- | $1.20 \times 10^{0}$ |
| **C6** | Isododecane | -- | -- | $1.22 \times 10^{0}$ |
| **C7** | 2-methylundecenoate | -- | -- | $1.89 \times 10^{0}$ |
| **C8** | Octamethylcyclotetrasiloxane | -- | -- | $2.24 \times 10^{0}$ |

(continued)

| Example | Oil | Thickening Polymer ("TP") | Conc. TP in Oil (wt%) | Viscosity (mPa·s) |
|---|---|---|---|---|
| **C9** | Hemisqualane[c] | -- | -- | $2.42 \times 10^0$ |
| **C10** | XIAMETER™ PMX-200 Silicone Fluid, 5 cSt[d] | -- | -- | $4.60 \times 10^0$ |
| **C11** | 2-butyl-1-octanol[e] | -- | -- | $1.91 \times 10^2$ |
| **C12** | DOWSIL™ 4-2737 Fluid[f] | -- | -- | $4.47 \times 10^1$ |
| **C13** | Sunflower Oil (not claimed) | -- | -- | $1.55 \times 10^2$ |
| **10** | XIAMETER™ PMX-200 Silicone Fluid, 0.65 cSt[a] | **Example S2** | 2.5 | $2.38 \times 10^2$ |
| **11** | Cetiol Ultimate[b] | **Example S2** | 2.5 | $9.49 \times 10^2$ |
| **12** | Isododecane | **Example S2** | 2.5 | $3.91 \times 10^2$ |
| **13** | 2-methylundecenoate | **Example S2** | 2.5 | $1.81 \times 10^3$ |
| **14** | Octamethylcyclotetrasiloxane | **Example S2** | 2.5 | $3.14 \times 10^3$ |
| **15** | Hemisqualane[c] | **Example S2** | 2.5 | $1.63 \times 10^3$ |
| **16** | XIAMETER™ MPX-200 Silicone Fluid, 5 cSt[d] | **Example S2** | 2.5 | $4.97 \times 10^2$ |
| **17** | 2-butyl-1-octanol[e] | **Example S2** | 2.5 | Gel |
| **18** | DOWSIL™ 4-2737 Fluid[f] | **Example S2** | 2.5 | B |
| **19** | Sunflower Oil (not claimed) | **Example S2** | 2.5 | B |
| **20** | Cetiol Ultimate[b] | **Example S2** | 5.0 | $4.73 \times 10^4$ |
| **21** | Isododecane | **Example S2** | 5.0 | $4.10 \times 10^4$ |
| **22** | 2-methylundecenoate | **Example S2** | 5.0 | $5.33 \times 10^4$ |
| **23** | Hemisqualane[c] | **Example S2** | 5.0 | $2.25 \times 10^4$ |
| **24** | Cetiol Ultimate[b] | **Example S1** | 8.0 | $4.35 \times 10^4$ |
| **25** | Hemisqualane[c] | **Example S1** | 8.0 | $1.19 \times 10^5$ |
| **26** | Isododecane | **Example S1** | 8.0 | $3.30 \times 10^4$ |
| **27** | 2-butyl-1-octanol[e] | **Example S1** | 8.0 | $1.52 \times 10^5$ |
| **28** | 2-methylundecenoate | **Example S1** | 8.0 | $8.34 \times 10^3$ |

[a] available from The Dow Chemical Company
[b] INCI: Undecane (and) tridecane available from BASF
[c] Fully saturated $C_{15}$ hydrocarbons (99%) available from Sigma-Aldrich
[d] available from The Dow Chemical Company
[e] Isofol 12 available from Sasol
[f] Linear siloxane fluid available from The Dow Chemical Company
B - inhomogeneous with suspended chunks

**Claims**

1. An oil/polymer blend comprising:

(a) 75 to 99.9 wt%, based on the weight of the oil/polymer blend, of an oil, wherein the oil is a cosmetically acceptable oil, selected from the group consisting of linear or branched $C_{8-30}$ alkanes, $C_{8-30}$ alkyl esters, $C_{8-30}$ carboxylic acid esters, $C_{8-30}$ linear or branched alcohols, silicone fluids, and mixtures thereof;
(b) 0.1 to 25 wt%, based on the weight of the oil/polymer blend, of a thickening polymer, wherein the thickening polymer is a silylated cellulose polymer, comprising a cellulose polymer functionalized with $-Si(R^1)_3$ groups; wherein each $R^1$ is independently selected from the group consisting of a hydrogen, a $C_{1-8}$ alkyl group, a $C_{1-8}$

haloalkyl group, an aryl group, a $C_{1-8}$ alkylaryl group and a $C_{1-8}$ haloalkylaryl group;

wherein the unfunctionalized cellulose polymer has a weight average molecular weight of 50,000 to 1,500,000 Daltons as measured by gel permeation chromatography (GPC).

**2.** The oil/polymer blend of claim 1, wherein the silylated cellulose polymer is of formula I

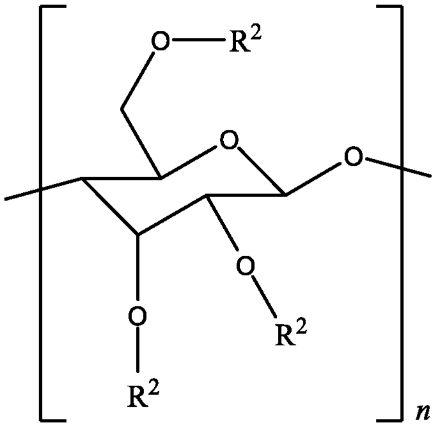

wherein $n$ is an average of 308 to 9,252; wherein each $R^2$ is independently selected from the group consisting of a hydrogen, a $C_{1-8}$ alkyl group and a $-Si(R^1)_3$ group; wherein each $R^1$ is independently selected from the group consisting of a hydrogen, a $C_{1-8}$ alkyl group, a $C_{1-8}$ haloalkyl group, an aryl group, a $C_{1-8}$ alkylaryl group and a $C_{1-8}$ haloalkylaryl group.

**3.** A skin care formulation, comprising:

a cosmetically acceptable carrier;
a color ingredient, wherein the color ingredient is selected from the group consisting of inorganic pigments, organic pigments, dyes, aqueous pigment dispersions and mixtures thereof; and
an oil/polymer blend of claim 1.

**4.** The skin care formulation of claim 3, wherein the color ingredient is selected from the group consisting of Ext. D&C Yellow No. 2, Ext. D & C Violet No. 2, FD&C Red No. 4, FD&C Red No. 40, FD&C Yellow No. 5, FD&C Yellow No. 6, FD&C Green No. 3, FD&C Blue No. 1, D&C Yellow No. 7, D&C Yellow No. 8, D&C Yellow No. 10, D&C Yellow No. 11, D&C Violet No. 2, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 34, D&C Red No. 33, D&C Red No. 36, D&C Green No. 5, D&C Green No. 6, D&C Green No. 8, D&C Blue No. 4, D&C Orange No. 4, D&C Orange No. 5, D&C Orange No. 10, D&C Orange No. 11, D&C Brown No. 1, Aluminum powder, Annatto, Bismuth citrate, Bismuth Oxychloride, Bronze powder, Caramel, Carmine, β-Carotene, Chromium hydroxide green, Chromium oxide green, Copper chlorophyllin, Copper powder, Dihydroxyacetone, Ferric Ammonium ferrocyanide, Ferric ferrocyanide, Guanine, Iron oxide, Manganese Violet, Mica, Silver, Titanium Dioxide, Ultramarine, Zinc Oxide and mixtures thereof.

**5.** The skin care formulation of claim 3, wherein the color ingredient includes an iron oxide and titanium dioxide.

**6.** A cosmetic method, comprising:

providing a skin care formulation of claim 3, and
applying the skin care formulation to skin.

**Patentansprüche**

**1.** Öl-/Polymer-Mischung, umfassend:

(a) zu 75 bis 99,9 Gew.-%, bezogen auf das Gewicht der Öl-/Polymer-Mischung, ein Öl,

wobei das Öl ein kosmetisch verträgliches Öl ist, ausgewählt aus der Gruppe bestehend aus linearen oder verzweigten $C_{8-30}$-Alkanen, $C_{8-30}$-Alkylestern, $C_{8-30}$-Carbonsäureestern, $C_{8-30}$-linearen oder -verzweigten Alkoholen, Silikonflüssigkeiten und Mischungen davon;

(b) zu 0,1 bis 25 Gew.-%, bezogen auf das Gewicht der Öl-/Polymer-Mischung, ein Verdickungspolymer, wobei das Verdickungspolymer ein silyliertes Cellulosepolymer ist, das ein mit -$Si(R^1)_3$-Gruppen funktionalisiertes Cellulosepolymer umfasst; wobei jedes $R^1$ unabhängig ausgewählt ist aus der Gruppe bestehend aus einem Wasserstoff, einer $C_{1-8}$-Alkylgruppe, einer $C_{1-8}$-Haloalkylgruppe, einer Arylgruppe, einer $C_{1-8}$-Alkylarylgruppe und einer $C_{1-8}$-Haloalkylarylgruppe;

wobei das unfunktionalisierte Cellulosepolymer ein gewichtsdurchschnittliches Molekulargewicht von 50.000 bis 1.500.000 Dalton aufweist, gemessen durch Gelpermeationschromatographie (GPC).

2. Öl-/Polymer-Mischung nach Anspruch 1, wobei das silylierte Cellulosepolymer die Formel I aufweist

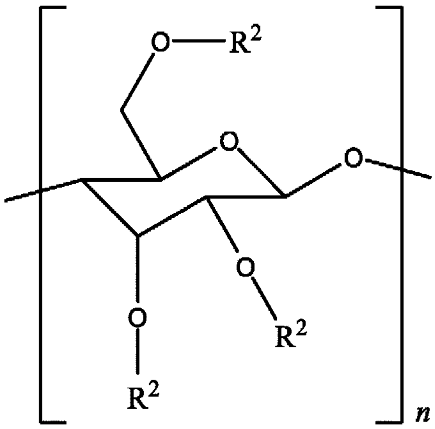

wobei *n* ein Durchschnitt von 308 bis 9.252 ist; wobei jedes $R^2$ unabhängig aus der Gruppe ausgewählt ist, bestehend aus einem Wasserstoff, einer $C_{1-8}$-Alkylgruppe und einer -$Si(R^1)_3$-Gruppe; wobei jedes $R^1$ unabhängig ausgewählt ist aus der Gruppe, bestehend aus einem Wasserstoff, einer $C_{1-8}$-Alkylgruppe, einer $C_{1-8}$-Haloalkylgruppe, einer Arylgruppe, einer $C_{1-8}$-Alkylarylgruppe und einer $C_{1-8}$-Haloalkylarylgruppe.

3. Hautpflegeformulierung, umfassend:

einen kosmetisch verträglichen Träger;

einen Farbbestandteil, wobei der Farbbestandteil aus der Gruppe ausgewählt ist, die aus anorganischen Pigmenten, organischen Pigmenten, Farbstoffen, wässrigen Pigmentdispersionen und Mischungen davon besteht; und

eine Öl-/Polymer-Mischung nach Anspruch 1.

4. Hautpflegeformulierung nach Anspruch 3, wobei der Farbbestandteil aus der Gruppe ausgewählt ist, bestehend aus Ext. D&C Gelb Nr. 2, Ext. D & C Violett Nr. 2, FD&C Rot Nr. 4, FD&C Rot Nr. 40, FD&C Gelb Nr. 5, FD&C Gelb Nr. 6, FD&C Grün Nr. 3, FD&C Blau Nr. 1, D&C Gelb Nr. 7, D&C Gelb Nr. 8, D&C Gelb Nr. 10, D&C Gelb Nr. 11, D&C Violett Nr. 2, D&C Rot Nr. 6, D&C Rot Nr. 7, D&C Rot Nr. 17, D&C Rot Nr. 21, D&C Rot Nr. 22, D&C Rot Nr. 27, D&C Rot Nr. 28, D&C Rot Nr. 30, D&C Rot Nr. 31, D&C Rot Nr. 34, D&C Rot Nr. 33, D&C Rot Nr. 36, D&C Grün Nr. 5, D&C Grün Nr. 6, D&C Grün Nr. 8, D&C Blau Nr. 4, D&C Orange Nr. 4, D&C Orange Nr. 5, D&C Orange Nr. 10, D&C Orange Nr. 11, D&C Braun Nr. 1, Aluminiumpulver, Annatto, Wismutcitrat, Wismutoxychlorid, Bronzepulver, Karamell, Carmin, β-Carotin, Chromhydroxidgrün, Chromoxidgrün, Kupferchlorophyllin, Kupferpulver, Dihydroxyaceton, Eisen(III)-ammoniumferrocyanid, Eisen(III)-ferrocyanid, Guanin, Eisenoxid, Manganviolett, Glimmer, Silber, Titandioxid, Ultramarin, Zinkoxid und Mischungen davon.

5. Hautpflegeformulierung nach Anspruch 3, wobei der Farbstoff ein Eisenoxid und Titandioxid einschließt.

6. Kosmetisches Verfahren, umfassend:

Bereitstellen einer Hautpflegeformulierung nach Anspruch 3 und

Auftragen der Hautpflegeformulierung auf die Haut.

## Revendications

1. Mélange huile/polymère comprenant :

   (a) 75 à 99,9 % en poids, sur la base du poids du mélange huile/polymère, d'une huile,
   dans lequel l'huile est une huile cosmétiquement acceptable, choisie dans le groupe constitué d'alcanes en $C_{8-30}$ linéaires ou ramifiés, d'esters d'alkyle en $C_{8-30}$, d'esters d'acide carboxylique en $C_{8-30}$, d'alcools en $C_{8-30}$ linéaires ou ramifiés, de fluides siliconés, et mélanges de ceux-ci ;
   (b) 0,1 à 25 % en poids, sur la base du poids du mélange huile/polymère, d'un polymère épaississant, dans lequel le polymère épaississant est un polymère de cellulose silylé, comprenant un polymère de cellulose fonctionnalisé par des groupes $-Si(R^1)_3$ ; dans lequel chaque $R^1$ est choisi indépendamment dans le groupe constitué d'un hydrogène, d'un groupe alkyle en $C_{1-8}$, d'un groupe haloalkyle en $C_{1-8}$, d'un groupe aryle, d'un groupe alkylaryle en $C_{1-8}$ et d'un groupe haloalkylaryle en $C_{1-8}$ ;
   dans lequel le polymère de cellulose non fonctionnalisé a une masse moléculaire moyenne en poids de 50 000 à 1 500 000 daltons, mesurée par chromatographie par perméation de gel (GPC).

2. Mélange huile/polymère selon la revendication 1, dans lequel le polymère de cellulose silylé est de formule I

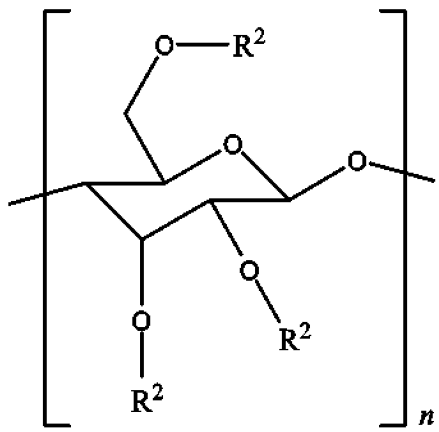

   dans lequel *n* est une moyenne de 308 à 9 252 ; dans lequel chaque $R^2$ est choisi indépendamment dans le groupe constitué d'un hydrogène, d'un groupe alkyle en $C_{1-8}$ et d'un groupe $-Si(R^1)_3$ ; dans lequel chaque $R^1$ est choisi indépendamment dans le groupe constitué d'un hydrogène, d'un groupe alkyle en $C_{1-8}$, d'un groupe haloalkyle en $C_{1-8}$, d'un groupe aryle, d'un groupe alkylaryle en $C_{1-8}$ et d'un groupe haloalkylaryle en $C_{1-8}$.

3. Formulation pour soins de la peau, comprenant :

   un support cosmétiquement acceptable ;
   un ingrédient colorant, dans laquelle l'ingrédient colorant est choisi dans le groupe constitué de pigments inorganiques, pigments organiques, colorants, dispersions aqueuses de pigments et mélanges de ceux-ci ; et
   un mélange huile/polymère selon la revendication 1.

4. Formulation pour soins de la peau selon la revendication 3, dans laquelle l'ingrédient colorant est choisi dans le groupe constitué de Ext. D&C jaune n° 2, Ext. D&C violet n° 2, FD&C rouge n° 4, FD&C rouge n° 40, FD&C jaune n° 5, FD&C jaune n° 6, FD&C vert n° 3, FD&C bleu n° 1, D&C jaune n° 7, D&C jaune n° 8, D&C jaune n° 10, D&C jaune n° 11, D&C violet n° 2, D&C rouge n° 6, D&C rouge n° 7, D&C rouge n° 17, D&C rouge n° 21, D&C rouge n° 22, D&C rouge n° 27, D&C rouge n° 28, D&C rouge n° 30, D&C rouge n° 31, D&C rouge n° 34, D&C rouge n° 33, D&C rouge n° 36, D&C vert n° 5, D&C vert n° 6, D&C vert n° 8, D&C bleu n° 4, D&C orange n° 4, D&C orange n° 5, D&C orange n° 10, D&C orange n° 11, D&C brun n° 1, poudre d'aluminium, rocou, citrate de bismuth, oxychlorure de bismuth, poudre de bronze, caramel, carmin, β-carotène, hydroxyde de chrome vert, oxyde de chrome vert, chlorophylline de cuivre, poudre de cuivre, dihydroxyacétone, ferrocyanure d'ammonium ferrique, ferrocyanure ferrique, guanine, oxyde de fer, violet de manganèse, mica, argent, dioxyde de titane, outremer, oxyde de zinc et mélanges de ceux-ci.

5. Formulation pour soins de la peau selon la revendication 3, dans laquelle l'ingrédient colorant comporte un oxyde de fer et un dioxyde de titane.

**6.** Procédé cosmétique, comprenant :

la fourniture d'une formulation pour soins de la peau selon la revendication 3, et
l'application sur la peau de la formulation pour soins de la peau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6060072 A, Konik
- US 20010021387, Krammer


### Non-patent literature cited in the description


- **STRIEGEL et al.** Modern Size Exclusion Liquid Chromatography: Practice of Gel Permeation and Gel Filtration Chromatography. John Wiley & Sons, 2009